# EUROPEAN PATENT SPECIFICATION

(11) **EP 3 787 584 B1**
(45) Date of publication and mention of the grant of the patent: **07.08.2024**
(21) Application number: 19727171.1
(22) Date of filing: 02.05.2019
(51) Int. Cl.: A61B 5/11, G08B 21/20, A61F 13/42, A61B 5/00, G08B 21/02

(54) **MONITORING SYSTEM FOR PROVIDING BOTH VISUAL AND NON-VISUAL DATA**
ÜBERWACHUNGSSYSTEM ZUR BEREITSTELLUNG VON VISUELLEN UND NICHTVISUELLEN DATEN
SYSTÈME DE SURVEILLANCE POUR FOURNIR DES DONNÉES VISUELLES ET NON VISUELLES

(30) Priority: 04.05.2018 US 201862666989 P; 27.08.2018 US 201862723179 P; 30.11.2018 US 201862773401 P
(43) Date of publication of application: 10.03.2021
(73) Proprietor: The Procter & Gamble Company, Cincinnati, OH 45202 (US)
(72) Inventor: ARIZTI, Bianca, 65824 Schwalbach am Taunus (DE); REIDY, Mark, 1213 Geneva (CH); SHER, Omer, 1213 Geneva (CH); HARRINGTON, Jennifer Mary, San Francisco, California 94080 (US)
(74) Representative: P&G Patent Germany
(86) International application number: PCT/US2019/030361
(87) International publication number: WO 2019/213370

(56) References cited:
- WO-A1-2015/127062
- US-A1- 2014 121 473
- US-A1- 2016 287 073

## Description

### FIELD OF THE DISCLOSURE

The present disclosure relates to systems that include both visual and non-visual data to monitor the well-being of individuals, such as infants and patients.

### BACKGROUND

Smart devices and systems for monitoring the care of infants, ageing adults, and patients are becoming commonplace. Camera and audio based baby monitors can be used to determine if an infant is sleeping or awake, content or upset, and the general nature of the infant via visual observation. However these monitors generally do not yield quantitative data regarding sleep, activity level and patterns thereof. Other monitoring devices associated with a diaper, such as those described in U.S. Patent No. 8,628,506, can be used to determine the location, body position, and physical activity of the diaper wearer. In addition to the '506 patent being silent regarding a camera, a skilled artisan reviewing this patent can likely conclude visual data is unneeded with the scope of non-visual data available by the disclosed devices. However, a caregiver, particularly a parent of an infant, can have an emotional need to be able to see his/her infant with their own eyes and/or hear the infant with their own ears even if a device associated with the infant's diaper or clothing provides non-visual data in all of the categories the parent is interested in.

US2014/121473 and US2016/287073 disclose systems for monitoring a patient.

Monitoring systems of the present disclosure have the capability to provide both visual and non-visual data about the nature of a monitored individual.

### SUMMARY

Monitoring systems of the present disclosure have the capability to provide visual and non-visual data about the nature of a monitored individual. The invention relates to a monitoring system as claimed in claim 1.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a schematic showing an exemplary monitoring system comprising a wearable sensor device and a camera device. Fig. 1 also shows a peripheral device that can be used to display and monitor data and other information collected by the wearable sensor device and/or camera device.
Fig. 2 is a schematic showing a wearable sensor device attached to an exterior of a diaper.
Fig. 3 is a front view of an exemplary camera device suitable for use in monitoring systems provided herein.
Fig. 4 is side view of an exemplary camera device that includes a camera and a camera docking station.
Fig. 5 is a perspective view of an exemplary camera device that is attached to a first docking station along with a second docking station for placement in a second location.
Fig. 6 is a diagram of an exemplary monitoring system comprising a data gathering module, a data processing module, and a communication module.
Fig. 7 is a flow diagram showing one operational scheme for monitoring systems of the present disclosure.
Fig. 8 is a partial view of an exemplary wearable sensor device of the present disclosure.
Fig. 9 is a caregiver-facing side of a wearable sensor device of the present disclosure.
Fig. 10 is an exemplary kit of the present disclosure, comprising a plurality of disposable absorbent articles, two wearable sensor devices, a camera, and two camera docking stations.

### DETAILED DESCRIPTION

Various non-limiting aspects of the present disclosure will now be described to provide an overall understanding of the principles of the structure, function, manufacture, and/or use of the subject matter disclosed herein. In the following detailed description, reference is made to the accompanying figures, which form a part hereof. In the figures, similar symbols can identify similar elements, unless context dictates otherwise. It will be readily understood that the aspects of the present disclosure, as generally described herein, and illustrated in the figures, can be arranged, substituted, combined, separated, and designed in a wide variety of different configurations.

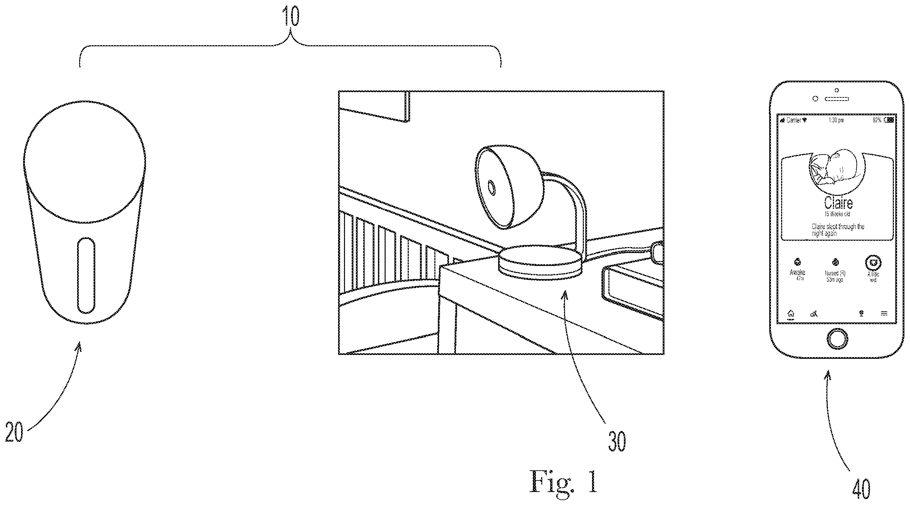

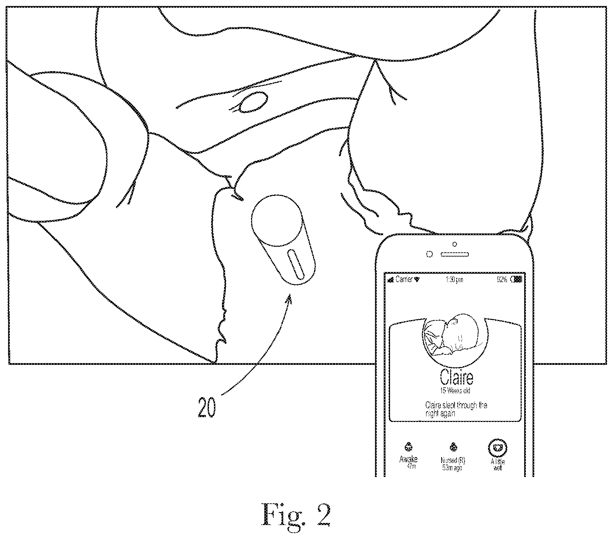

Monitoring systems of the present disclosure have the capability to provide both visual and non-visual data about the nature of a monitored individual. With reference to Fig. 1, an exemplary monitoring system 10 is shown that comprises a wearable sensor device 20 to generate non-visual data and a camera device 30 to generate visual data and optionally audio. Wearable sensor device 20 can be associated with a disposable absorbent article (see, e.g., Fig. 2) or clothing that is worn by an infant or patient being monitored. Wearable sensor device 20 can be a single-use sensor or a multi-use sensor that can be removed from clothing or a soiled disposable absorbent article and associated with a freshly-donned article. A multi-component wearable sensor device is also contemplated, wherein a first sensor component (e.g., capacitor) forms a part of the manufactured article and a second, multi-use sensor component (e.g., power source and data transmitter) connects with the first sensor component to create sensor functionality.

A peripheral device 40 in the form of a smart phone is shown in Fig. 1, as an example of a device that can be used by a caregiver to observe and manage the non-visual and/or visual data or aspects thereof that are provided by monitoring systems of the present disclosure. The non-visual data transmitted from wearable sensor device 20 can be communicated to camera device 30 and thereafter communicated to a peripheral device, or alternatively, be communicated to the peripheral device bypassing camera device 30. Camera device 30 can also be configured to communicate non-visual data from wearable sensor device 20 in the absence of communicating visual data obtained by camera device 30.

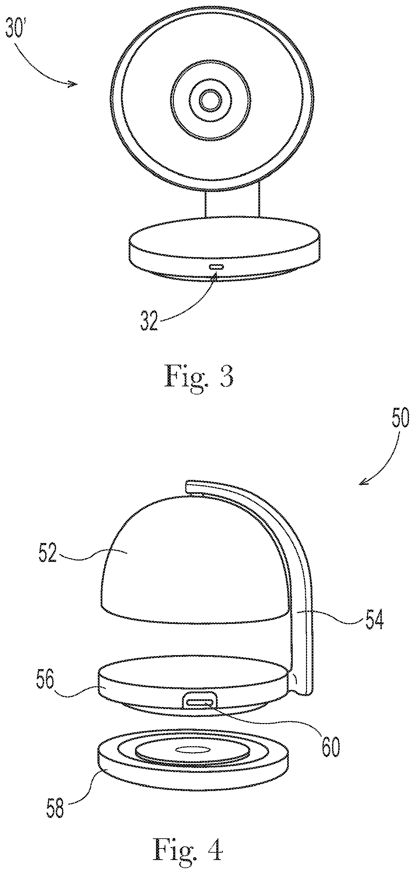

Monitoring systems of the present disclosure include additional sensors beyond wearable sensor device 20. An environmental sensor to measure environmental conditions where an infant or patient is located is used. The environmental sensors include a temperature sensor, a relative humidity sensor, a carbon monoxide sensor, a VOC (volatile organic compound) sensor, a smoke sensor, a motion sensor, and combinations thereof. The camera device comprises these additional sensors. By way of example only, Fig. 3 shows a camera device 30' that includes a sensor port 32 that allows a temperature sensor and/or relative humidity sensor disposed within camera device 30' to measure such environmental conditions.

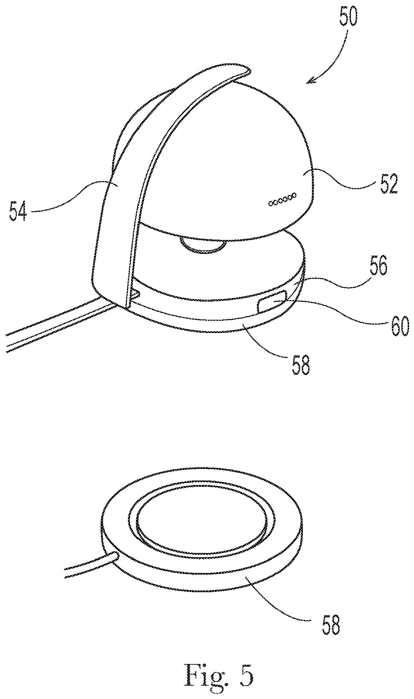

Camera devices forming part of the disclosed monitoring systems can take many forms and can include various functionality, including an ability to capture video and still visual images, contain night vision technology to capture visual images in a dark or semi-dark environment, contain noise and/or motion activation features, and ability to transmit current and historical visual information. An exemplary camera device 50, shown in Figs. 4 and 5, includes a camera head 52 pivotally connected to an arm 54 that is attached to a support member in the form of a base 56. Support members of suitable camera devices can take many forms and can include a variety of features for securing the camera device to its intended structure or location. Having a pivotable or otherwise moveable camera head can provide numerous benefits, including, for example, being able to capture visual data from different fields of view without moving the camera base position. In some forms, the gap between camera head 52 and arm 54 or base 56 can be between about 3 mm and 16.5 mm to help mitigate pinching and entrapment. As can be seen in Fig. 4, camera head 52 can also pivot downward to face base 56 for privacy and to prevent capturing unintentional visual data.

Exemplary camera device 50 further includes a docking station 58 that is releasably engageable with base 56. A latch 60 is employed to positively secure base 56 to docking station 58. Other means, for example, magnets, can equally be used for making the secure connection between base 56 and docking station 58. The monitoring systems can include multiple docking stations, as shown in Fig. 5, to enable capturing visual data from multiple locations with a single camera. The multiple docking stations can be similar or dissimilar to one another. For example, one of the multiple docking stations can be configured (e.g., with brackets to secure the docking station power cord to a wall) for a more permanent location such as a nursery, while a second docking station is configured for mobility to be taken to different locations within or outside of one's home. The skilled artisan should readily appreciate that multiple standalone camera devices can be included with monitoring systems disclosed herein rather than having a single camera and multiple docking stations.

The camera devices of the monitoring systems herein can include light sources and audio capability. The light sources can emit constant or changing light effects. The light source can, for example, emit light outside of the visible blue light spectrum (e.g., wavelengths in the range of 300 nm to 700 nm, or 400 nm to 500 nm). Blue light hazard function weight radiance (L_{B}) can be less than 0.2. The audio can be one-way or two-way. The light sources can avoid blinking and flashing LEDs with frequency between 3 Hz and 60 Hz to help mitigate seizures in photosensitive individuals (both monitored individuals and caregivers). The audio capability can permit communication between a caregiver and a monitored infant or patient, including from the infant/patient to the caregiver. The audio capability can also provide caregiver communications, music or other soothing sounds.

The camera devices of the monitoring systems herein can also include a variety of safety features. A mount of the camera device and/or secure positioning within a docking station should be able to sustain certain loading before the camera device or camera within the docking station is dislodged. For example, the placed positioning can withstand three times the device or component weight applied downwardly for 1 minute and/or 50 newtons applied sideways for 1 minute (after stress relief testing) according to IEC 60065-14 wall mounting test (section 19.7). The camera device with or without a separable docking station can be sufficiently light to help mitigate harm to a monitored individual or caregiver should it accidently fall on them. Along these lines, the camera device (with or without a docking station) can weigh less than 500, 400, 300, 200, 100, or even 75 grams.

Power cords that are extremely flexible can more easily become wrapped around an undesirable object such as a monitored individual. To help prevent this, the power cord of the camera devices can have a safe release mechanism or easy cord pull (e.g., under 0.65 pound force) which can help mitigate strangulation concerns. Increasing the bending stiffness and/or its cross-sectional areas may also mitigate strangulation hazards.

The wearable sensor device can be a single-use device, or a multi-use device. The wearable sensor device can be attachable to skin (via hydrogel or bio-adhesive material, for example), to a disposable article such as an absorbent article, and/or to clothing worn by a monitored individual. Various attachment mechanisms can be employed for attaching the wearable sensor device to an article of clothing or disposable absorbent article such as a diaper, pant, pad or brief. For example, hook and loop fastening mechanisms, magnets, adhesives, thermal bonds, and male and female mating fasteners such as snaps and buttons. Receiving features, such as pockets, recesses, and voids can also be employed that essentially hold the wearable sensor device with or without attachment features. In yet another form, an auxiliary article can be used to integrate the wearable sensor device with a disposable absorbent article. The auxiliary article can be in the form of a pant-like reusable garment designed to fit over a disposable absorbent article.

In one form, the wearable sensor device is adapted for attachment to an outer cover of a disposable absorbent article. Hook and loop features can be used with this attachment approach. For example, a strip of hook material can be affixed to one surface of the sensor housing, where the hooks can engage directly with material used for the outer cover or with an added strip of "loop" material.

The wearable sensor device can include a sensor housing that generally protects sensors and other electronic components disposed therein, as well as inhibiting unwanted contact of the same with an infant, patient, or caregiver. The housing can be made from a variety of materials, both flexible and rigid, examples of which include thermoplastic polymers, thermoplastic elastomers, silicone, Tecaform, Tecanant, thermoplastic copolyester (TPC) and combinations thereof. Other materials can also be employed for the housing so long as it is generally regarded as safe for human contact and does not cause irritation or other unwanted health effects. The materials can include bio-compatible, medical grade, and non-cytotoxic materials. Inclusion of a bittering agent or other approaches can optionally be used to discourage placement of the sensor device in one's mouth or otherwise tampering with the wearable sensor device.

The wearable sensor device comprises a plurality of electronic component disposed on and/or within the housing. Typically, the electronic components include at least one sensor, a transmitter, and a power source (e.g., a disposable battery or a rechargeable battery). The number and type of sensors employed by the sensor device are chosen based on the application of the monitoring systems disclosed herein.

The monitoring systems are capable of capturing and communicating a combination of visual data and non-visual data to caregiver. With connection back to the background section above, one exemplary monitoring system of the present disclosure includes the combination of a wearable sensor device comprising a motion sensor and a camera device. The motion sensor can provide non-visual sleep-related data including, for example, time of sleep, sleep duration, sleep position, sleep position changes, and activity-related data including, for example, tummy time, level of activity, and nursing/feeding. Suitable motion sensors include accelerometers, inertial measurement units (IMUs), gyroscopes, and magnetometers. As noted in the background, notwithstanding the benefit of obtaining the non-visual sleep-related data, a caregiver wants/needs to see a monitored individual to understand visual sleep (or pre- and/or post-sleep) aspects of a monitored individual. The camera device can provide this visual confirmation desired by the caregiver. Having both data sources available additionally provides comfort to a caregiver when the camera device is unable to capture information regarding the monitored individual. For example, the camera device is powered off or the monitored individual is not within the field of view of the camera. In this scenario the wearable sensor device can provide data and information to the caregiver until the camera device is able to capture the monitored individual.

Besides obtaining sleep-related data and information, monitoring systems provided herein are capable of obtaining both visual and non-visual data and information related to other activities that contain a motion aspect, including, for example, eating, drinking, breastfeeding, walking, crawling, and tummy-time. The non-visual data and information can be determined by a wearable sensor device or a remote sensor device (unattached to a monitored individual or anything worn by the individual) that is capable of measuring motion and a camera device.

Some monitoring systems of the present disclosure can include a wearable sensor device comprising two or more sensors and a camera device. The wearable sensor device is associated with a disposable absorbent article and comprises a first sensor that is capable of capturing non-visual data and information regarding a monitored individual, and a second sensor that is capable of capturing a condition (e.g., clean or soiled with body exudates) of the disposable absorbent article. The first sensor associated with the wearable sensor device can be a motion sensor as described above for capturing information about sleep or activity aspects. The second sensor can be a wetness sensor or bowel movement (BM) sensor for detecting urine or feces within the disposable absorbent article, and/or track absorbent article usage. The wearable sensor device can include both a wetness sensor and a BM sensor in some forms. In one form, the wearable sensor device is free of audio capturing or communicating components.

Wetness sensors for detecting the presence of urine or other bodily fluid can include optical sensors, color sensors, and electrical sensors that comprise a resistance, capacitance, inductance or continuity sensitive indicator. A resistance sensitive indicator can be provided, for example, by providing two electrical conductors disposed at a given spatial distance relative to each other. A VOC sensor is one suitable type of a BM sensor. The VOC sensors can be of MOS-type (metal oxide). BM sensors can be capable of acting as an electronic nose to detect chemical signatures of organic materials associated with body exudates, including, for example, skatole, mercaptans, amines, volatile fatty acids, acetone, methyl acetate, and methanol. BM sensors can also include an optical or color sensor to detect the presence of feces in the article. Along these lines, multiple optical or color sensors can be used to detect both urine and feces, based either on their inherent colors or based on use of an indicator that changes color in the presence of urine and/or feces. For example, the following enzymes associated with body exudates can trigger an optical change in an included indicator that can be sensed by an optical or color sensor: urease, trypsin, chemotrypsin, LAP, lipase, amilase, and urease.

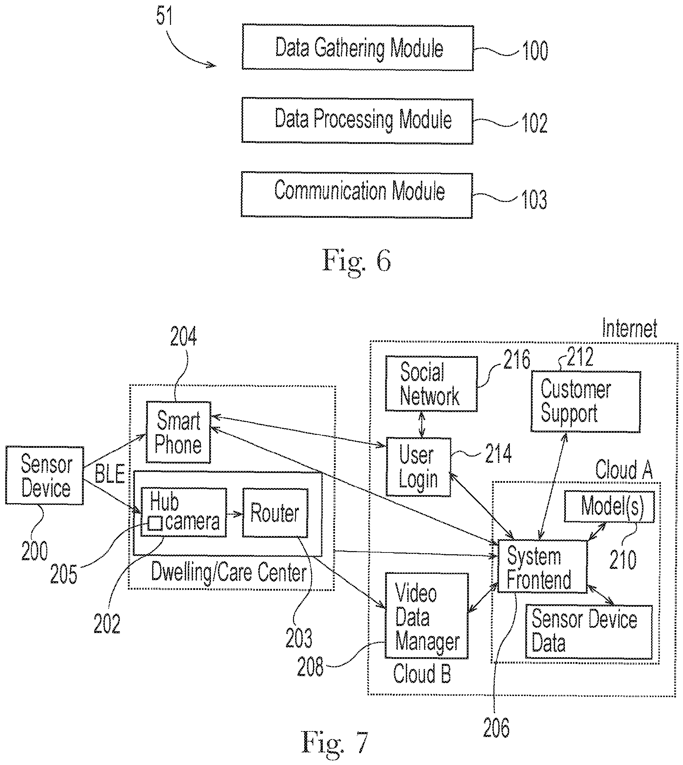

With reference to Fig. 6, monitoring systems S1 of the present disclosure can include three elements: a data gathering module 100, a data processing module 102, and a communication module 103. The data gathering module can include a camera device 202/205 and a wearable sensor device 200 and that can be associated with a disposable absorbent article or article of clothing worn by an infant or patient. Besides the wearable sensor device and camera device, the data gathering module can comprise other sensor devices or equipment in proximity to the person wearing the sensor device. For example, the data gathering module can comprise an environmental sensor for sensing smoke, carbon monoxide, VOC's, temperature, relative humidity; a motion sensor, an audio recorder, and the like.

The data processing module can comprise data transmission, data storage, data interpretation, and/or data manipulation to transform the data from the data gathering module into consumer understandable information related to the wellbeing of an individual, including, for example, feeding, sleeping, and/or voiding. And the communication module comprises a software application for communicating (e.g., displaying) the data and information from the wearable sensor device and the camera device.

Fig. 7 is an exemplary schematic illustrating how monitoring systems of the present disclosure can operate. Two example operation modes are shown; one where the wearable sensor device 200 is worn by an infant/patient that is located within a dwelling or care center 202 with wireless communication (e.g., Bluetooth low energy, 15.4, ad hoc mesh networks, and the like) conducted between wearable sensor device 200 and a hub 202 comprising a camera device 205. And another where the infant/patient is located remotely (e.g., in a car or stroller) with wireless communication conducted between wearable sensor device 200 and a smart peripheral device 204 (e.g., phone). Data and information from wearable sensor device 200 is communicated via a router 203 or smart device 204 to a system frontend 206 for transforming the data and information to consumer usable information provided via a software application.

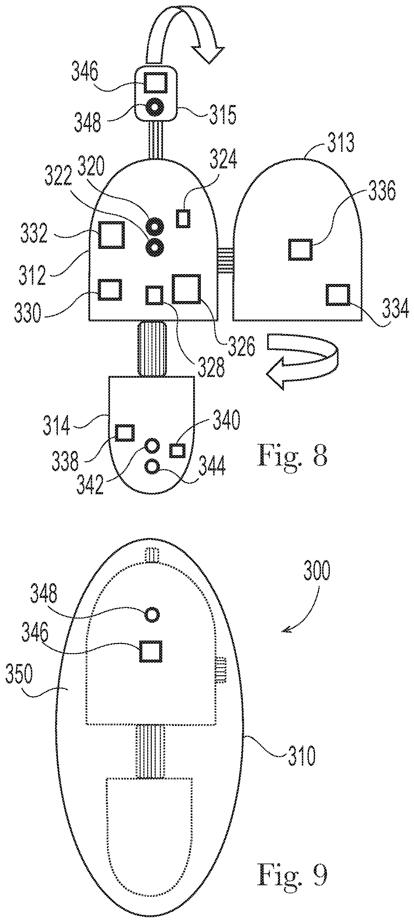

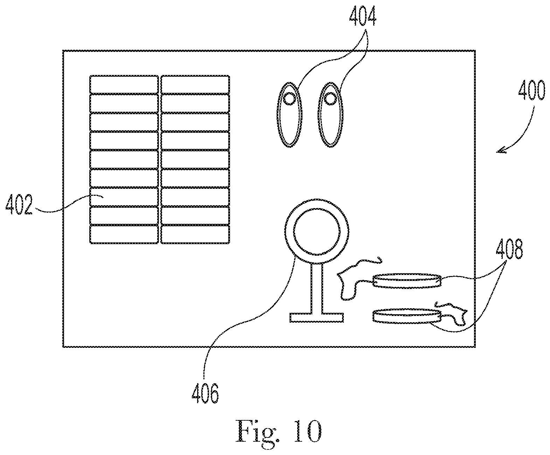

Figs. 8 and 9 illustrate aspects of one exemplary wearable sensor device 300 comprising a plurality of sensors and other electronic components. Fig. 8 shows the wearable sensor device in a manufactured form before its final configuration and encasing with a sensor housing 310 (shown in Fig. 10). With reference to Fig. 8, multiple electrically-connected printed circuit boards 312, 313, 314, and 315 are employed. Printed circuit board 312 comprises an optical sensor 320, an absorbent article-facing light source 322, a power management component 324, a flash memory component 326, an optical sensor frontend 328, a processor and transmitter component 330, and an antenna 332. Printed circuit board 313 comprises a motion sensor (e.g., accelerometer) 334 and a power source (e.g., coin cell battery) 336. Printed circuit board 314 comprises a BM sensor (e.g., VOC sensor) 338, a temperature and relative humidity sensor 340, a second optical sensor 342, and a second absorbent article-facing light source 344. And printed circuit board 315 comprises a consumer-engageable button 346 for activating or otherwise operating sensor device 300, and a caregiver-facing light source 348 to indicate an operational aspect of sensor device 300. Button 346 or similar engageable feature can be used for multiple tasks. For example, button 346 can be initially activated for "waking" the wearable sensor device up from a power-save mode and/or manually activated by a caregiver upon changing an absorbent article if a wearer of the article has had a bowel movement. Acknowledgement of a bowel movement via action of button 346 or similar engageable feature can be communicated by the wearable sensor device to a communication module for tracking timing, frequency, or other aspects of a wearer's bowel movement history. One skilled in the art would appreciate that a single circuit board can be employed in a sensor device, as well as other numbers of circuit boards beyond what is shown in Fig. 8.

In one form, the wearable sensor device comprises a power source in the form of a battery, a transmitter, multiple optical sensors (e.g., a color sensor), multiple light sources (e.g., an LED), and an accelerometer. The wearable sensor device is attached to an absorbent article comprising a wetness indicator, as described above, such that the light source can direct light onto the wetness indicator. The wetness indicator changes appearance (e.g., changes color) when a wearer urinates into the absorbent article. The optical sensor measures the reflected light from the wetness indicator to sense when a urination event occurs. Multiple pairs of absorbent article-facing light sources and optical sensors can be employed to sense changes of a wetness indicator at different points along the indicator to confirm a urination event has occurred, or predict the volume of urine and/or number of urination events that occurred. A signal from the optical sensor can then be transmitted to the data processing module. The accelerometer is employed to track data associated with sleep and awake times. The awake data can include awake feeding motion data and awake non-feeding motion. The accelerometer is capable of sensing breastfeeding times and provide feeding information for one's right breast and left breast. The accelerometer can also be configured to sense bottle feeding aspects.

The data processing module can comprise data transmission, data storage, data interpretation, data filtering, and/or data manipulation to transform the data from the data gathering module into consumer understandable information related to the wellbeing of an individual, including, for example, feeding, sleeping, and/or voiding. The data processing module can include algorithms to parse/filter the received data. Data processing can be accomplished by one or more devices and in the same or different locations. For example, the wearable sensor device can optionally employ a memory device to temporarily store data. One reason for temporary storage of data is when communication between the wearable sensor device and a remote data processing module component and/or the information communication module is unavailable.

The wearable sensor device can also optionally employ a data processor for processing raw data from one or more sensors associated with the wearable sensor device prior to transmitting data/information based on the raw data. This can reduce the volume of data/information transmitted from the wearable sensor device, and thereby reduce the amount of power required and accompanying electromagnetic radiation emission.

The communication module comprises a software application operable on a computer device to display information related to the data obtained by the data gathering module, including data transformed via the data processing module. The computer device can be a smart phone, as is shown in Fig. 1, but other computer devices, such as a laptop, tablet, digital assistant (ALEXA and GOOGLE HOME, for example) can be used to communicate information to one or more caregivers. Monitoring systems of the present disclosure can provide both visual and non-visual data and information regarding a monitored individual. Software applications are typically employed to display and otherwise communicate this data and information. While separate software applications can be employed to view and/or manage the visual and non-visual components, respectively, it is preferred for a single software application to be utilized to both provide visual data of the monitored individual and provide the non-visual data and information arising from a wearable sensor device and a camera device. Data and information from the wearable sensor device and/or camera device can be encrypted in the event someone other than the caregiver gains access to the data and information.

Wearable sensor devices and monitoring systems including the same can form a part of consumer purchasable kit. One exemplary kit includes two or more wearable sensor devices as described herein, a camera device, a plurality of absorbent articles that can accept the wearable sensor devices, and access to a software application for viewing data and information flowing from the wearable sensor device and camera device. A subscription can be offered to consumers that provides delivery of additional absorbent articles, wearable sensor devices, and/or continued access and operation of the software application. For example, a subscription can include an automatic delivery of a number of absorbent articles every two weeks along with a code or other mechanism for continued operation of the software application. The subscription can work with an affirmative action request by a consumer or as an automatic delivery order that delivers products on a set reoccurring schedule until the schedule reaches a predetermined endpoint, or is altered or discontinued by the consumer. Similar to the subscription example above, packages of absorbent articles can be sold in brick and mortar locations wherein the packages contain a code for operation of the software application to view data and information received from a wearable sensor device according to the present disclosure.

Fig. 10 shows an exemplary kit 400 comprising a plurality of absorbent articles 402, two wearable sensor devices 404, a camera 406, and two camera docking stations 408.

While the discussion has focused on infants and patients, systems of the present invention are also applicable for elderly care. The sensors associated with such systems can be capable of association with an elderly's skin, durable undergarments, disposable absorbent articles, bed materials, bed pads, and/or clothing articles.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular forms of the present disclosure have been illustrated and described, it would be obvious to those skilled in the art that various other changes and modifications can be made without departing from the scope of the disclosure. It should be understood that other forms can include more or less of each element shown in a given figure. Further, some of the illustrated elements can be combined or omitted. Yet further, an exemplary form can include elements that are not illustrated in the figures. The various aspects and forms disclosed herein are for purposes of illustration and are not intended to be limiting, with the true scope being indicated by the following claims.

## Claims

1. A monitoring system, comprising:
a. a multi-use wearable sensor device temporarily associated with an absorbent article, the multi-use wearable sensor device comprising a first sensor capable of capturing non-visual data or information regarding a monitored individual and a second sensor capable of capturing a condition of the absorbent article, wherein the non-visual data or information regarding a monitored individual and/or condition of the absorbent article defines wearable data; and
b. a camera device capable of capturing visual information regarding the monitored individual, wherein the camera device comprises one or more environmental sensors, the environmental sensor measuring environmental conditions where an infant or patient is located and wherein the environmental sensor is selected from the group consisting of a temperature sensor, a relative humidity sensor, a carbon monoxide sensor, a VOC (volatile organic compound) sensor, a smoke sensor, a motion sensor, and combinations thereof.

2. The monitoring system of claim 1, wherein the first sensor comprises an accelerometer.

3. The monitoring system of any of the preceding claims, wherein the second sensor is a wetness sensor.

4. The monitoring system of claim 1, wherein the one or more environmental sensors comprises a temperature sensor.

5. The monitoring system of claim 1, wherein the one or more environmental sensors comprises a relative humidity sensor.

6. The monitoring system of any of the preceding claims, wherein the camera device comprises a camera, a data receiver for receiving at least some of the wearable data, and a transmitter for communicating the wearable data and/or visual information captured by the camera.

7. The monitoring system of claim 6, wherein the camera device is capable of communicating the wearable data without communication of any of the visual information to a peripheral device monitorable by a caregiver.

8. The monitoring system of claim 6, wherein the multi-use wearable sensor device is capable of bypassing communication with the camera device and communicating the wearable data to a peripheral device monitorable by a caregiver.

9. The monitoring system of any of the preceding claims, wherein the camera device comprises a camera; a first docking station for temporarily receiving the camera; and a second docking station for temporarily receiving the camera.

10. The monitoring system of any of the preceding claims, wherein the camera device comprises a camera that is moveable from a first position comprising a first field of view for monitoring the well-being of a person to a second position having a second field of view that is different from the first field of view to enable privacy.

## Patentansprüche

1. Überwachungssystem, umfassend:
a. eine tragbare Mehrweg-Sensorvorrichtung, die mit einem Absorptionsartikel vorübergehend verbunden ist, die tragbare Mehrweg-Sensorvorrichtung umfassend einen ersten Sensor, der in der Lage ist, nichtvisuelle Daten oder Informationen bezüglich einer überwachten Person zu erfassen, und einen zweiten Sensor, der in der Lage ist, einen Zustand des absorbierenden Absorptionsartikels zu erfassen, wobei die nicht-visuellen Daten oder Informationen bezüglich einer überwachten Person und/oder des Zustands des Absorptionsartikels tragbare Daten definieren; und
b. eine Kameravorrichtung, die in der Lage ist, visuelle Informationen bezüglich der überwachten Person zu erfassen, wobei die Kameravorrichtung einen oder mehrere Umgebungssensoren umfasst, wobei der Umgebungssensor den Umgebungszustand misst, wo sich ein Kleinkind oder ein Patient befindet, und wobei der Umgebungssensor aus der Gruppe ausgewählt ist, bestehend aus einem Temperatursensor, einem Sensor für relative Feuchtigkeit, einem Kohlenmonoxidsensor, einem VOC-Sensor (flüchtige organische Verbindungen), einem Rauchsensor, einem Bewegungssensor und Kombinationen davon.

2. Überwachungssystem nach Anspruch 1, wobei der Sensor einen Beschleunigungsmesser umfasst.

3. Überwachungssystem nach einem der vorstehenden Ansprüche, wobei der zweite Sensor ein Nässesensor ist.

4. Überwachungssystem nach Anspruch 1, wobei der eine oder die mehreren Umgebungssensoren einen Temperatursensor umfassen.

5. Überwachungssystem nach Anspruch 1, wobei der eine oder die mehreren Umgebungssensoren einen Sensor für relative Feuchtigkeit umfassen.

6. Überwachungssystem nach einem der vorstehenden Ansprüche, wobei die Kameravorrichtung eine Kamera, einen Datenempfänger zum Empfangen wenigstens einiger der tragbaren Daten und einen Sender zum Übermitteln der tragbaren Daten und/oder visuellen Informationen, die durch die Kamera erfasst werden, umfasst.

7. Überwachungssystem nach Anspruch 6, wobei die Kameravorrichtung in der Lage ist, die tragbaren Daten ohne Übermittlung von beliebigen der visuellen Informationen an eine periphere Vorrichtung, die durch eine Pflegekraft überwachbar ist, zu übermitteln.

8. Überwachungssystem nach Anspruch 6, wobei die tragbare Mehrweg-Sensorvorrichtung in der Lage ist, die Übermittlung mit der Kameravorrichtung zu umgehen und die tragbaren Daten an eine periphere Vorrichtung, die durch eine Pflegekraft überwachbar ist, zu übermitteln.

9. Überwachungssystem nach einem der vorstehenden Ansprüche, wobei die Kameravorrichtung eine Kamera; eine erste Dockingstation zum vorübergehenden Empfangen der Kamera; und eine zweite Dockingstation zum vorübergehenden Empfangen der Kamera umfasst.

10. Überwachungssystem nach einem der vorstehenden Ansprüche, wobei die Kameravorrichtung eine Kamera umfasst, die von einer ersten Position, umfassend ein erstes Sichtfeld zum Überwachen des Wohlbefindens einer Person, in eine zweite Position, die ein zweites Sichtfeld aufweist, das sich vom ersten Sichtfeld unterscheidet, um Privatsphäre zu ermöglichen, bewegbar ist.

## Revendications

1. Système de surveillance comprenant :
a. un dispositif capteur portable à usage multiple associé temporairement à un article absorbant, le dispositif capteur portable à usage multiple comprenant un premier capteur capable de capturer des données non visuelles ou des informations concernant un individu surveillé et un second capteur capable de capturer un état de l'article absorbant, dans lequel les données non visuelles ou informations concernant un individu surveillé et/ou un état de l'article absorbant définissent des données portables ; et
b. un dispositif de caméra capable de capturer des informations visuelles concernant l'individu surveillé, dans lequel le dispositif de caméra comprend un ou plusieurs capteurs environnementaux, le capteur environnemental mesurant des conditions environnementales où un nourrisson ou un patient est situé et dans lequel le capteur environnemental est choisi dans le groupe constitué d'un capteur de température, d'un capteur d'humidité relative, d'un capteur de monoxyde de carbone, d'un capteur de COV (composé organique volatil), d'un capteur de fumée, d'un capteur de mouvement et de combinaisons de ceux-ci.

2. Système de surveillance selon la revendication 1, dans lequel le premier capteur comprend un accéléromètre.

3. Système de surveillance selon l'une quelconque des revendications précédentes, dans lequel le second capteur est un capteur d'humidité.

4. Système de surveillance selon la revendication 1, dans lequel les un ou plusieurs capteurs environnementaux comprennent un capteur de température.

5. Système de surveillance selon la revendication 1, dans lequel les un ou plusieurs capteurs environnementaux comprennent un capteur d'humidité relative.

6. Système de surveillance selon l'une quelconque des revendications précédentes, dans lequel le dispositif de caméra comprend une caméra, un récepteur de données pour recevoir au moins certaines des données portables, et un émetteur pour communiquer les données portable et/ou les informations visuelles capturées par la caméra.

7. Système de surveillance selon la revendication 6, dans lequel le dispositif de caméra est capable de communiquer les données portables sans communication de l'une quelconque des informations visuelles à un dispositif périphérique pouvant être surveillé par un prestataire de soins.

8. Système de surveillance selon la revendication 6, dans lequel le dispositif de capteur portable à usage multiple est capable de contourner une communication avec le dispositif de caméra et de communiquer les données portables à un dispositif périphérique pouvant être surveillé par un prestataire de soins.

9. Système de surveillance selon l'une quelconque des revendications précédentes, dans lequel le dispositif de caméra comprend une caméra ; une première station d'accueil pour recevoir temporairement la caméra ; et une seconde station d'accueil pour recevoir temporairement la caméra.

10. Système de surveillance selon l'une quelconque des revendications précédentes, dans lequel le dispositif de caméra comprend une caméra qui est mobile depuis une première position comprenant un premier champ de vision pour surveiller le bien-être d'une personne à une seconde position ayant un second champ de vision qui est différent du premier champ de vision pour permettre la confidentialité.
